(19) Europäisches Patentamt
European Patent Office
Office européen des brevets

(11) **EP 2 732 803 B1**

(12) **EUROPÄISCHE PATENTSCHRIFT**

(45) Veröffentlichungstag und Bekanntmachung des Hinweises auf die Patenterteilung:
**25.04.2018 Patentblatt 2018/17**

(51) Int Cl.:
*A61K 8/11* *(2006.01)*     *C09B 67/02* *(2006.01)*
*C11D 3/50* *(2006.01)*     *A61Q 19/00* *(2006.01)*
*A61K 8/02* *(2006.01)*

(21) Anmeldenummer: **12007807.6**

(22) Anmeldetag: **19.11.2012**

(54) **Thermisch öffnende stabile Kern/Schale-Mikrokapseln**

Thermally opening stable core/shell micro capsules

Microcapsules à noyau/coquille stables à ouverture thermique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**21.05.2014 Patentblatt 2014/21**

(73) Patentinhaber: **Follmann GmbH & Co. KG**
**32423 Minden (DE)**

(72) Erfinder: **Last Klaus**
**38124 Braunschweig (DE)**

(74) Vertreter: **von Renesse, Dorothea et al**
**König-Szynka-Tilmann-von Renesse**
**Patentanwälte Partnerschaft mbB**
**Postfach 11 09 46**
**40509 Düsseldorf (DE)**

(56) Entgegenhaltungen:
JP-A- 2005 115 194     US-A- 5 260 069
US-A- 6 143 276     US-A- 6 165 667
US-A1- 2012 128 747

**Beschreibung**

Die vorliegende Erfindung betrifft thermisch öffnende Mikrokapseln. Die vorliegende Erfindung betrifft insbesondere Mikrokapseln mit oberflächenmodifizierten Sprengmitteln.

Mikrokapseln, die als Kernmaterial feste, flüssige oder gasförmige Stoffe enthalten können, sind im Stand der Technik bekannt und haben in den letzten Jahren Einzug in eine Vielzahl von Anwendungen gefunden. Dabei ist insbesondere ihre Eigenschaft hervorzuheben, einen Wirkstoff für einige Zeit zu umschließen und anschließend freizusetzen. Dieses ist z. B. in der Wasch- und Reinigungsmittelindustrie von besonderem Interesse, in der Mittel hergestellt werden, um Textilien mit einem lang anhaltenden Duft zu versehen. Eine Mikroverkapselung der Duftstoffe stellt sicher, dass diese über einen gewissen Zeitraum freigesetzt werden.

Die möglichst kontrollierte Freisetzung der Inhalte (Kernmaterialien) von Kapseln ist auch in anderen Bereichen von höchstem Interesse wie z.B. bei selbstheilenden Materialien, bei der Nahrungsmittelkonservierung oder der Freisetzung von Pharmazeutika oder Katalysatoren. Es wurden bislang verschiedene Methoden der Verkapselung von Wirkstoffen und deren Freisetzung entwickelt. Aus dem Bereich der Pharmazie ist seit langem bekannt, dass Kapseln nach Einnahme durch den Patienten im Verdauungstrakt zersetzt werden und so die Wirkstoffe freisetzen. Diese Kapseln können Sprengmittel enthalten, die im Verdauungstrakt Flüssigkeit aufnehmen, dadurch stark anschwellen und die Kapselhülle mechanisch zerstören.

Bekannt sind außerdem chemische Arten der Freisetzung, so z.B. auf dem Gebiet des Korrosionsschutzes.

Äußere Einflüsse führen zu einer Zersetzung über beispielsweise eine Depolymerisierung der Kapselhülle und somit zu einer Freisetzung der Inhaltsstoffe der Kapsel. Auch photoinduzierte Öffnungen von Kapseln sind im Stand der Technik beschrieben. Dazu gehören die gezielte Zerstörung der Kapselhülle wie etwa mit einem Laser, das Auslösen der Depolymerisation der Kapselhülle oder das Verdampfen der Inhaltsstoffe, das zu einem Sprengen der Kapselhülle führt. Auch elektrische Reize zum Öffnen von Kapselwänden sind auf dem Gebiet von selbstheilenden elektronischen Bauteilen und Stromkreisen bekannt. Dieses bedingt allerdings die Notwendigkeit des Einbaus von hochfunktionellen Gruppen oder Monomeren in die polymere Kapselhülle. Diese Notwendigkeit besteht auch auf dem Gebiet der magnetischen Öffnung von Mikrokapseln durch den Einbau von magnetisch anregbaren Funktionalitäten, Molekülen oder Partikel auf Nanobauteilgröße. Auch thermische Öffnungen sind bekannt, z.B. durch das Auslösen des Schrumpfens der Kapselhülle, durch thermische Zersetzung, oder durch Sprengen der Hülle durch einen Druckanstieg, der durch das Verdampfen des flüchtigen Kerninhalts ausgelöst wird. Ein solches thermisch ausgelöstes Freisetzen von Inhaltsstoffen findet beispielsweise in der Freisetzung von Duftstoffen oder desodorierenden Stoffen in der Kosmetik Anwendung.

Nachteilig bei allen diesen Öffnungsmechanismen ist allerdings, dass diese immer eine enge Abstimmung von Kapselcharakteristika wie Wandstärke, Vernetzungsdichte, Permeabilität, chemische Zusammensetzung, mechanische Eigenschaften, Kapselgröße, Kapselumfeld und Kapselinhalt notwendig machen. Zudem entsprechen diese Kapselsysteme nicht den in der Industrie verwendeten stabilen Kern/Schale-Systemen, welche ihre Kernmaterialien aufgrund ihrer besonderen Dichtigkeit und chemischen Resistenz über lange Zeiträume vor dem Austritt schützen. Bei Öffnungsmechanismen wie der chemischen oder elektrischen Öffnung, der Öffnung durch Lichtreize und durch chemische Reize macht man sich unter anderem die Änderung von Konformationen in Azofarbstoffen, die Spaltung von Disulfidbrücken oder Acetaten, die Depolymerisation der Kapselwand durch Spaltung von Carbamaten oder Lipidbrücken durch Enzyme oder pH-Wert-Änderungen zunutze. Allerdings bedingt dieses die Notwendigkeit des Einbaus von Funktionen in das Wandmaterial, das dadurch beispielsweise hinsichtlich seiner Netzwerkdichte negativ beeinflusst wird. Zudem wird für eine schnelle Öffnung eine hohe Funktionsdichte benötigt. Ähnlich verhält es sich bei einer thermomechanischen Öffnung durch elektrische oder magnetische Reize. Hier ist der Einbau von metallhaltigen Nanopartikeln in die Kapselwand bekannt, die durch das Anlegen eines Magnetfeldes oder elektrischen Feldes zu Oszillationen angeregt werden, wodurch Hitze erzeugt und so die Kapselwand zerstört wird. Nachteilig dabei ist der dadurch bedingte hohe Energieeintrag, sowie die Notwendigkeit von teuren speziellen Apparaturen und Anlagen, mit denen die Öffnung der Kapseln und damit die Freisetzung der Inhaltsstoffe bewirkt werden kann.

Die einfachsten Methoden zur Öffnung von Mikrokapseln sind die rein mechanisch basierten Systeme, wie etwa das Zerdrücken oder Zerquetschen der Kapseln.

Von besonderem Interesse ist die Öffnung der Mikrokapseln durch thermische Reize, da sie besser zu steuern und zu dosieren ist und auch bei Mikrokapseln angewendet werden kann, die sich in einer Lösung oder Dispersion oder in oder auf Lebewesen befinden.

Aus dem Stand der Technik sind Partikel bekannt, die beim Anlegen von thermischen Reizen eine Expansion erfahren. So beschreibt beispielsweise die KR-A-2005/0084965 einen thermisch expandierbaren Partikel, umfassend eine polymere Hülle und einen flüchtigen Inhaltsstoff, der bei einer Temperatur unterhalb des Weichpunktes des Polymers in die Gasphase übergeht. Nicht gelehrt wird die gezielte Freisetzung von Inhaltsstoffen bei einer bestimmten Temperatur, sondern lediglich die thermische induzierte Expansion einer Mikrokapsel.

In der WO-A-2010/014011 wird ein Partikel offenbart, der eine polymere Hülle aufweist und ein mit Wasser aufquellendes Sprengmittel enthält, welches bei physiologischen Temperaturen und pH-Werten Wasser aufnimmt und nach Einnahme des Partikels durch den Patienten in dessen Verdauungstrakt die Kapsel öffnet und somit deren Inhaltsstoffe freisetzen kann.

W. Wang et al. (Microfluidic Preparation of Multicompartment Microcapsules for Co-Encapsulation and Controlled Release of Multiple Components, Posterabstract, 19. Oktober 2011, Minneapolis Convention Center) beschreibt einen Partikel mit mehreren verkapselten Ölkernen und einer Hülle, die sich bei einer Temperaturerhöhung zusammenzieht, dabei aufreisst und so die Ölkerne freisetzt. Auch eine Modifizierung der Hülle für eine Schrumpfung bei Änderung des pH-Wertes oder bei Zufuhr von anderen externen Stimuli wie beispielsweise Glucose wird beschrieben.

Dokument JP 2005 115194 A offenbart einen Toner in Form von Mikrokapseln, deren Kern ein Sprengmittel enthält und die in einem vorbestimmten Temperaturbereich öffnen. Aus dem Stand der Technik ist bisher noch keine Mikrokapsel bekannt, die innerhalb eines engen Temperaturbereiches reproduzierbar bei einer hohen Entladung ihres Inhaltes öffnet und gleichzeitig den hohen Anforderungen an ein industriell einsetzbares System erfüllt. Die Anforderungen an ein solches System sind unter anderem geringe Kosten, Flexibilität hinsichtlich der Anpassung an Kundenwünsche, Anwendungen und zu verwendende Materialien, eine Scale-up-Fähigkeit, ein günstiges Preis-Leistungsverhältnis und somit eine hohe Wirtschaftlichkeit, die Erfüllung von Stabilitätsgarantien und generelle Einfachheit des Systems.

Vor diesem Hintergrund bestand die Aufgabe der vorliegenden Erfindung darin, eine verbesserte thermisch öffnende Mikrokapsel bereitzustellen, die in mindestens einem dieser Kriterien eine Verbesserung gegenüber dem Stand der Technik erzielt. Insbesondere sollte sich die Kapselwand innerhalb eines engen Temperaturbereichs öffnen und den Kapselinhalt schnell innerhalb eines engen Zeitraums freigeben.

Es wurde nun überraschend gefunden, dass diese Aufgabe durch eine Mikrokapsel enthaltend ein oberflächenmodifiziertes Sprengmittel gelöst werden kann.

Ein Gegenstand der vorliegenden Erfindung ist damit eine Mikrokapsel umfassend eine Schale und einen Kern, der mindestens einen Wirkstoff und mindestens ein oberflächenmodifiziertes Sprengmittel umfasst, wobei das Sprengmittel ein expandierbarer Partikel ist mit einer Partikelhülle und einem Partikelkern, dadurch gekennzeichnet, dass die Oberfläche der Partikelhülle hydrophobisiert ist. Als Material für die Kapselwände der vorliegenden Erfindung können dem Fachmann insbesondere bekannte Materialien wie beispielsweise Phenoplast-Polymere, Melamin-Formaldehyd-Polymere, Polyurethan, Gelatine, Polyamide oder Polyharnstoffe verwendet werden. Diese Kapselsysteme zeigen eine hohe chemische und physikalische Resistenz und bilden stabilste und dichteste Mikrokapseln aus.

Bevorzugte Kapselwände für die erfindungsgemäßen Kapseln sind dem Fachmann beispielsweise aus der WO-A-2011/110368 bekannt und umfassen bzw. bestehen aus einem Amin, einem Aldehyd und gegebenenfalls einem (Meth)acrylat-AMPS bzw. -AMPP-Copolymer. Dabei versteht man unter AMPS die 2-Acrylamido-2-methyl-propansulfonsäure oder dessen Salze und unter AMPP die 2-Acrylamido-2-methyl-propanphosphonsäure oder dessen Salze. Solche Beispiele für bevorzugte Kapselwände sind:

Phloroglucin, Glutardialdehyd, AMPS/Hydroxyethylmethacrylat-Copolymer;
Phloroglucin, Succindialaldehyd, AMPS/Hydroxyethylmethacrylat-Copolymer;
Phloroglucin, Glyoxal, AMPS/Hydroxyethylmethacrylat-Copolymer;
Phloroglucin, Glyoxylsäure, AMPS/Hydroxyethylmethacrylat-Copolymer;
Phloroglucin, Glutardialdehyd, AMPS/Hydroxyethylacrylat-Copolymer;
Phloroglucin, Succindialaldehyd, AMPS/Hydroxyethylacrylat-Copolymer;
Phloroglucin, Glyoxal, AMPS/Hydroxyethylacrylat-Copolymer;
Phloroglucin, Glyoxylsäure, AMPS/Hydroxyethylacrylat-Copolymer;
Phloroglucin, Glutardialdehyd, AMPS/Hydroxypropylmethacrylat-Copolymer;
Phloroglucin, Succindialaldehyd, AMPS/Hydroxypropylmethacrylat-Copolymer;
Phloroglucin, Glyoxal, AMPS/Hydroxypropylmethacrylat-Copolymer;
Phloroglucin, Glyoxylsäure, AMPS/Hydroxypropylmethacrylat-Copolymer;
Phloroglucin, Glutardialdehyd, AMPS/Hydroxypropylacrylat-Copolymer;
Phloroglucin, Succindialaldehyd, AMPS/Hydroxypropylacrylat-Copolymer;
Phloroglucin, Glyoxal, AMPS/Hydroxypropylacrylat-Copolymer;
Phloroglucin, Glyoxylsäure, AMPS/Hydroxypropylacrylat-Copolymer;
Phloroglucin, Glutardialdehyd, AMPS/Hydroxybutylmethacrylat-Copolymer;
Phloroglucin, Succindialaldehyd, AMPS/Hydroxybutylmethacrylat-Copolymer;
Phloroglucin, Glyoxal, AMPS/Hydroxybutylmethacrylat-Copolymer;
Phloroglucin, Glyoxylsäure, AMPS/Hydroxbutylmethacrylat-Copolymer;
Phloroglucin, Glutardialdehyd, AMPS/Hydroxybutylacrylat-Copolymer;
Phloroglucin, Succindialaldehyd, AMPS/Hydroxybutylacrylat-Copolymer;
Phloroglucin, Glyoxal, AMPS/Hydroxybutylacrylat-Copolymer;
Phloroglucin, Glyoxylsäure, AMPS/Hydroxybutylacrylat-Copolymer;
Phloroglucin, Glutardialdehyd, AMPS/Polyethylenglykolmonomethacrylat-Copolymer;

Phloroglucin, Succindialaldehyd, AMPS/Polyethylenglykolmonomethacrylat-Copolymer;
Phloroglucin, Glyoxal, AMPS/ Polyethylenglykolmonomethacrylat-Copolymer;
Phloroglucin, Glyoxylsäure, AMPS/Polyethylenglykolmonomethacrylat-Copolymer;
Phloroglucin, Glutardialdehyd, AMPS/Polyethylenglykolmonoacrylat-Copolymer;
Phloroglucin, Succindialaldehyd, AMPS/Polyethylenglykolmonoacrylat-Copolymer;
Phloroglucin, Glyoxal, AMPS/ Polyethylenglykolmonoacrylat-Copolymer;
Phloroglucin, Glyoxylsäure, AMPS/ Polyethylenglykolmonoacrylat-Copolymer;
Phloroglucin, Glutardialdehyd, AMPS/Polypropylenglykolmonomethacrylat-Copolymer;
Phloroglucin, Succindialaldehyd, AMPS/Polypropylenglykolmonomethacrylat-Copolymer;
Phloroglucin, Glyoxal, AMPS/ Polypropylenglykolmonomethacrylat-Copolymer;
Phloroglucin, Glyoxylsäure, AMPS/ Polypropylenglykolmonomethacrylat-Copolymer;
Phloroglucin, Glutardialdehyd, AMPS/Polypropylenglykolmonoacrylat-Copolymer;
Phloroglucin, Succindialaldehyd, AMPS/Polypropylenglykolmonoacrylat-Copolymer;
Phloroglucin, Glyoxal, AMPS/ Polypropylenglykolmonoacrylat-Copolymer;
Phloroglucin, Glyoxylsäure, AMPS/ Polypropylenglykolmonoacrylat-Copolymer;
Phloroglucin, Glutardialdehyd, AMPS/Methoxy-polyethylenglykolmonomethacrylat-Copolymer;
Phloroglucin, Succindialaldehyd, AMPS/Methoxy-polyethylenglykolmonomethacrylat-Copolymer;
Phloroglucin, Glyoxal, AMPS/ Methoxy-polyethylenglykolmonomethacrylat-Copolymer;
Phloroglucin, Glyoxylsäure, AMPS/ Methoxy-polyethylenglykolmonomethacrylatCopolymer;
Phloroglucin, Glutardialdehyd, AMPS/Methoxy-polyethylenglykolmonoacrylat-Copolymer;
Phloroglucin, Succindialaldehyd, AMPS/Methoxy-polyethylenglykolmonoacrylat-Copolymer;
Phloroglucin, Glyoxal, AMPS/ Methoxy-polyethylenglykolmonoacrylat-Copolymer;
Phloroglucin, Glyoxylsäure, AMPS/ Methoxy-polyethylenglykolmonoacrylatCopolymer;
Resorcin, Glutardialdehyd, AMPS/Hydroxyethylmethacrylat-Copolymer;
Resorcin, Succindialaldehyd, AMPS/Hydroxyethylmethacrylat-Copolymer;
Resorcin, Glyoxal, AMPS/Hydroxyethylmethacrylat-Copolymer;
Resorcin, Glyoxylsäure, AMPS/Hydroxyethylmethacrylat-Copolymer
Resorcin, Glutardialdehyd, AMPS/Hydroxyethylacrylat-Copolymer;
Resorcin, Succindialaldehyd, AMPS/Hydroxyethylacrylat-Copolymer;
Resorcin, Glyoxal, AMPS/Hydroxyethylacrylat-Copolymer;
Resorcin, Glyoxylsäure, AMPS/Hydroxyethylacrylat-Copolymer
Resorcin, Glutardialdehyd, AMPS/Hydroxypropylmethacrylat-Copolymer;
Resorcin, Succindialaldehyd, AMPS/Hydroxypropylmethacrylat-Copolymer;
Resorcin, Glyoxal, AMPS/Hydroxypropylmethacrylat-Copolymer;
Resorcin, Glyoxylsäure, AMPS/Hydroxypropylmethacrylat-Copolymer
Resorcin, Glutardialdehyd, AMPS/Hydroxypropylacrylat-Copolymer;
Resorcin, Succindialaldehyd, AMPS/Hydroxypropylacrylat-Copolymer;
Resorcin, Glyoxal, AMPS/Hydroxypropylacrylat-Copolymer;
Resorcin, Glyoxylsäure, AMPS/Hydroxypropylacrylat-Copolymer
Resorcin, Glutardialdehyd, AMPS/Hydroxybutylmethacrylat-Copolymer;
Resorcin, Succindialaldehyd, AMPS/Hydroxybutylmethacrylat-Copolymer;
Resorcin, Glyoxal, AMPS/Hydroxybutylmethacrylat-Copolymer;
Resorcin, Glyoxylsäure, AMPS/Hydroxybutylmethacrylat-Copolymer
Resorcin, Glutardialdehyd, AMPS/Hydroxybutylacrylat-Copolymer;
Resorcin, Succindialaldehyd, AMPS/Hydroxybutylacrylat-Copolymer;
Resorcin, Glyoxal, AMPS/Hydroxybutylacrylat-Copolymer;
Resorcin, Glyoxylsäure, AMPS/Hydroxybutylacrylat-Copolymer
Resorcin, Glutardialdehyd, AMPS/Polyethylenglykolmonomethacrylat-Copolymer;
Resorcin, Succindialaldehyd, AMPS/Polyethylenglykolmonomethacrylat-Copolymer;
Resorcin, Glyoxal, AMPS/ Polyethylenglykolmonomethacrylat-Copolymer;
Resorcin, Glyoxylsäure, AMPS/ Polyethylenglykolmonomethacrylat-Copolymer;
Resorcin, Glutardialdehyd, AMPS/Polyethylenglykolmonoacrylat-Copolymer;
Resorcin, Succindialaldehyd, AMPS/Polyethylenglykolmonoacrylat-Copolymer;
Resorcin, Glyoxal, AMPS/ Polyethylenglykolmonoacrylat-Copolymer;
Resorcin, Glyoxylsäure, AMPS/ Polyethylenglykolmonoacrylat-Copolymer;
Resorcin, Glutardialdehyd, AMPS/Polypropylenglykolmonomethacrylat-Copolymer;
Resorcin, Succindialaldehyd, AMPS/Polypropylenglykolmonomethacrylat-Copolymer;
Resorcin, Glyoxal, AMPS/ Polypropylenglykolmonomethacrylat-Copolymer;

Resorcin, Glyoxylsäure, AMPS/ Polypropylenglykolmonomethacrylat-Copolymer;
Resorcin, Glutardialdehyd, AMPS/Polypropylenglykolmonoacrylat-Copolymer;
Resorcin, Succindialaldehyd, AMPS/Polypropylenglykolmonoacrylat-Copolymer;
Resorcin, Glyoxal, AMPS/ Polypropylenglykolmonoacrylat-Copolymer;
Resorcin, Glyoxylsäure, AMPS/ Polypropylenglykolmonoacrylat-Copolymer;
Resorcin, Glutardialdehyd, AMPS/Methoxy-polyethylenglykolmonomethacrylat-Copolymer;
Resorcin, Succindialaldehyd, AMPS/Methoxy-polyethylenglykolmonomethacrylat-Copolymer;
Resorcin, Glyoxal, AMPS/ Methoxy-polyethylenglykolmonomethacrylat-Copolymer;
Resorcin, Glyoxylsäure, AMPS/ Methoxy-polyethylenglykolmonomethacrylat-Copolymer;
Resorcin, Glutardialdehyd, AMPS/Methoxy-polyethylenglykolmonoacrylat-Copolymer;
Resorcin, Succindialaldehyd, AMPS/Methoxy-polyethylenglykolmonoacrylat-Copolymer;
Resorcin, Glyoxal, AMPS/ Methoxy-polyethylenglykolmonoacrylat-Copolymer;
Resorcin, Glyoxylsäure, AMPS/ Methoxy-polyethylenglykolmonoacrylat-Copolymer;
Harnstoff, Glutardialdehyd, AMPS/Hydroxyethylmethacrylat-Copolymer;
Harnstoff, Succindialaldehyd, AMPS/Hydroxyethylmethacrylat-Copolymer;
Harnstoff, Glyoxal, AMPS/Hydroxyethylmethacrylat-Copolymer;
Harnstoff, Glyoxylsäure, AMPS/Hydroxyethylmethacrylat-Copolymer
Harnstoff, Glutardialdehyd, AMPS/Hydroxyethylacrylat-Copolymer;
Harnstoff, Succindialaldehyd, AMPS/Hydroxyethylacrylat-Copolymer;
Harnstoff, Glyoxal, AMPS/Hydroxyethylacrylat-Copolymer;
Harnstoff, Glyoxylsäure, AMPS/Hydroxyethylacrylat-Copolymer
Harnstoff, Glutardialdehyd, AMPS/Hydroxypropylmethacrylat-Copolymer;
Harnstoff, Succindialaldehyd, AMPS/Hydroxypropylmethacrylat-Copolymer;
Harnstoff, Glyoxal, AMPS/Hydroxypropylmethacrylat-Copolymer;
Harnstoff, Glyoxylsäure, AMPS/Hydroxypropylmethacrylat-Copolymer
Harnstoff, Glutardialdehyd, AMPS/Hydroxypropylacrylat-Copolymer;
Harnstoff, Succindialaldehyd, AMPS/Hydroxypropylacrylat-Copolymer;
Harnstoff, Glyoxal, AMPS/Hydroxypropylacrylat-Copolymer;
Harnstoff, Glyoxylsäure, AMPS/Hydroxypropylacrylat-Copolymer
Harnstoff, Glutardialdehyd, AMPS/Hydroxybutylmethacrylat-Copolymer;
Harnstoff, Succindialaldehyd, AMPS/Hydroxybutylmethacrylat-Copolymer;
Harnstoff, Glyoxal, AMPS/Hydroxybutylmethacrylat-Copolymer;
Harnstoff, Glyoxylsäure, AMPS/Hydroxybutylmethacrylat-Copolymer
Harnstoff, Glutardialdehyd, AMPS/Hydroxybutylacrylat-Copolymer;
Harnstoff, Succindialaldehyd, AMPS/Hydroxybutylacrylat-Copolymer;
Harnstoff, Glyoxal, AMPS/Hydroxybutylacrylat-Copolymer;
Harnstoff, Glyoxylsäure, AMPS/Hydroxybutylacrylat-Copolymer
Harnstoff, Glutardialdehyd, AMPS/Polyethylenglykolmonomethacrylat-Copolymer;
Harnstoff, Succindialaldehyd, AMPS/Polyethylenglykolmonomethacrylat-Copolymer;
Harnstoff, Glyoxal, AMPS/ Polyethylenglykolmonomethacrylat-Copolymer;
Harnstoff, Glyoxylsäure, AMPS/ Polyethylenglykolmonomethacrylat-Copolymer;
Harnstoff, Glutardialdehyd, AMPS/Polyethylenglykolmonoacrylat-Copolymer;
Harnstoff, Succindialaldehyd, AMPS/Polyethylenglykolmonoacrylat-Copolymer;
Harnstoff, Glyoxal, AMPS/ Polyethylenglykolmonoacrylat-Copolymer;
Harnstoff, Glyoxylsäure, AMPS/ Polyethylenglykolmonoacrylat-Copolymer;
Harnstoff, Glutardialdehyd, AMPS/Polypropylenglykolmonomethacrylat-Copolymer;
Harnstoff, Succindialaldehyd, AMPS/Polypropylenglykolmonomethacrylat-Copolymer;
Harnstoff, Glyoxal, AMPS/ Polypropylenglykolmonomethacrylat-Copolymer;
Harnstoff, Glyoxylsäure, AMPS/ Polypropylenglykolmonomethacrylat-Copolymer;
Harnstoff, Glutardialdehyd, AMPS/Polypropylenglykolmonoacrylat-Copolymer;
Harnstoff, Succindialaldehyd, AMPS/Polypropylenglykolmonoacrylat-Copolymer;
Harnstoff, Glyoxal, AMPS/ Polypropylenglykolmonoacrylat-Copolymer;
Harnstoff, Glyoxylsäure, AMPS/ Polypropylenglykolmonoacrylat-Copolymer;
Harnstoff, Glutardialdehyd, AMPS/Methoxy-polyethylenglykolmonomethacrylat-Copolymer;
Harnstoff, Succindialaldehyd, AMPS/Methoxy-polyethylenglykolmonomethacrylat-Copolymer;
Harnstoff, Glyoxal, AMPS/ Methoxy-polyethylenglykolmonomethacrylat-Copolymer;
Harnstoff, Glyoxylsäure, AMPS/ Methoxy-polyethylenglykolmonomethacrylat-Copolymer;
Harnstoff, Glutardialdehyd, AMPS/Methoxy-polyethylenglykolmonoacrylat-Copolymer;

Harnstoff, Succindialaldehyd, AMPS/Methoxy-polyethylenglykolmonoacrylat-Copolymer;
Harnstoff, Glyoxal, AMPS/ Methoxy-polyethylenglykolmonoacrylat-Copolymer;
Harnstoff, Glyoxylsäure, AMPS/ Methoxy-polyethylenglykolmonoacrylat-Copolymer;
Melamin, Glutardialdehyd, AMPS/Hydroxyethylmethacrylat-Copolymer;
Melamin, Succindialaldehyd, AMPS/Hydroxyethylmethacrylat-Copolymer;
Melamin, Glyoxal, AMPS/Hydroxyethylmethacrylat-Copolymer;
Melamin, Glyoxylsäure, AMPS/Hydroxyethylmethacrylat-Copolymer
Melamin, Glutardialdehyd, AMPS/Hydroxyethylacrylat-Copolymer;
Melamin, Succindialaldehyd, AMPS/Hydroxyethylacrylat-Copolymer;
Melamin, Glyoxal, AMPS/Hydroxyethylacrylat-Copolymer;
Melamin, Glyoxylsäure, AMPS/Hydroxyethylacrylat-Copolymer
Melamin, Glutardialdehyd, AMPS/Hydroxypropylmethacrylat-Copolymer;
Melamin, Succindialaldehyd, AMPS/Hydroxypropylmethacrylat-Copolymer;
Melamin, Glyoxal, AMPS/Hydroxypropylmethacrylat-Copolymer;
Melamin, Glyoxylsäure, AMPS/Hydroxypropylmethacrylat-Copolymer
Melamin, Glutardialdehyd, AMPS/Hydroxypropylacrylat-Copolymer;
Melamin, Succindialaldehyd, AMPS/Hydroxypropylacrylat-Copolymer;
Melamin, Glyoxal, AMPS/Hydroxypropylacrylat-Copolymer;
Melamin, Glyoxylsäure, AMPS/Hydroxypropylacrylat-Copolymer
Melamin, Glutardialdehyd, AMPS/Hydroxybutylmethacrylat-Copolymer;
Melamin, Succindialaldehyd, AMPS/Hydroxybutylmethacrylat-Copolymer;
Melamin, Glyoxal, AMPS/Hydroxybutylmethacrylat-Copolymer;
Melamin, Glyoxylsäure, AMPS/Hydroxybutylmethacrylat-Copolymer
Melamin, Glutardialdehyd, AMPS/Hydroxybutylacrylat-Copolymer;
Melamin, Succindialaldehyd, AMPS/Hydroxybutylacrylat-Copolymer;
Melamin, Glyoxal, AMPS/Hydroxybutylacrylat-Copolymer;
Melamin, Glyoxylsäure, AMPS/Hydroxybutylacrylat-Copolymer
Melamin, Glutardialdehyd, AMPS/Polyethylenglykolmonomethacrylat-Copolymer;
Melamin, Succindialaldehyd, AMPS/Polyethylenglykolmonomethacrylat-Copolymer;
Melamin, Glyoxal, AMPS/ Polyethylenglykolmonomethacrylat-Copolymer;
Melamin, Glyoxylsäure, AMPS/ Polyethylenglykolmonomethacrylat-Copolymer;
Melamin, Glutardialdehyd, AMPS/Polyethylenglykolmonoacrylat-Copolymer;
Melamin, Succindialaldehyd, AMPS/Polyethylenglykolmonoacrylat-Copolymer;
Melamin, Glyoxal, AMPS/ Polyethylenglykolmonoacrylat-Copolymer;
Melamin, Glyoxylsäure, AMPS/ Polyethylenglykolmonoacrylat-Copolymer;
Melamin, Glutardialdehyd, AMPS/Polypropylenglykolmonomethacrylat-Copolymer;
Melamin, Succindialaldehyd, AMPS/Polypropylenglykolmonomethacrylat-Copolymer;
Melamin, Glyoxal, AMPS/ Polypropylenglykolmonomethacrylat-Copolymer;
Melamin, Glyoxylsäure, AMPS/ Polypropylenglykolmonomethacrylat-Copolymer;
Melamin, Glutardialdehyd, AMPS/Polypropylenglykolmonoacrylat-Copolymer;
Melamin, Succindialaldehyd, AMPS/Polypropylenglykolmonoacrylat-Copolymer;
Melamin, Glyoxal, AMPS/ Polypropylenglykolmonoacrylat-Copolymer;
Melamin, Glyoxylsäure, AMPS/ Polypropylenglykolmonoacrylat-Copolymer;
Melamin, Glutardialdehyd, AMPS/Methoxy-polyethylenglykolmonomethacrylat-Copolymer;
Melamin, Succindialaldehyd, AMPS/Methoxy-polyethylenglykolmonomethacrylat-Copolymer;
Melamin, Glyoxal, AMPS/ Methoxy-polyethylenglykolmonomethacrylat-Copolymer;
Melamin, Glyoxylsäure, AMPS/ Methoxy-polyethylenglykolmonomethacrylat-Copolymer;
Melamin, Glutardialdehyd, AMPS/Methoxy-polyethylenglykolmonoacrylat-Copolymer;
Melamin, Succindialaldehyd, AMPS/Methoxy-polyethylenglykolmonoacrylat-Copolymer;
Melamin, Glyoxal, AMPS/ Methoxy-polyethylenglykolmonoacrylat-Copolymer;
Melamin, Glyoxylsäure, AMPS/ Methoxy-polyethylenglykolmonoacrylat-Copolymer;
Resorcin, Glutardialdehyd, AMPP/Polyethylenglykolmonomethacrylat-Copolymer;
Resorcin, Succindialaldehyd, AMPP/Polyethylenglykolmonomethacrylat-Copolymer;
Resorcin, Glyoxal, AMPP/ Polyethylenglykolmonomethacrylat-Copolymer;
Resorcin, Glyoxylsäure, AMPS/ Polyethylenglykolmonomethacrylat-Copolymer;
Resorcin, Glutardialdehyd, AMPP/Polyethylenglykolmonoacrylat-Copolymer;
Resorcin, Succindialaldehyd, AMPP/Polyethylenglykolmonoacrylat-Copolymer;
Resorcin, Glyoxal, AMPP/ Polyethylenglykolmonoacrylat-Copolymer;

Resorcin, Glyoxylsäure, AMPP/ Polyethylenglykolmonoacrylat-Copolymer;
Resorcin, Glutardialdehyd, AMPP/Polypropylenglykolmonomethacrylat-Copolymer;
Resorcin, Succindialaldehyd, AMPP/Polypropylenglykolmonomethacrylat-Copolymer;
Resorcin, Glyoxal, AMPP/ Polypropylenglykolmonomethacrylat-Copolymer;
Resorcin, Glyoxylsäure, AMPP/ Polypropylenglykolmonomethacrylat-Copolymer;
Resorcin, Glutardialdehyd, AMPP/Polypropylenglykolmonoacrylat-Copolymer;
Resorcin, Succindialaldehyd, AMPP/Polypropylenglykolmonoacrylat-Copolymer;
Resorcin, Glyoxal, AMPP/ Polypropylenglykolmonoacrylat-Copolymer;
Resorcin, Glyoxylsäure, AMPP/ Polypropylenglykolmonoacrylat-Copolymer;
Resorcin, Glutardialdehyd, AMPP/Methoxy-polyethylenglykolmonomethacrylat-Copolymer;
Resorcin, Succindialaldehyd, AMPP/Methoxy-polyethylenglykolmonomethacrylat-Copolymer;
Resorcin, Glyoxal, AMPP/ Methoxy-polyethylenglykolmonomethacrylat-Copolymer;
Resorcin, Glyoxylsäure, AMPP/ Methoxy-polyethylenglykolmonomethacrylat-Copolymer;
Resorcin, Glutardialdehyd, AMPP/Methoxy-polyethylenglykolmonoacrylat-Copolymer;
Resorcin, Succindialaldehyd, AMPP/Methoxy-polyethylenglykolmonoacrylat-Copolymer;
Resorcin, Glyoxal, AMPP/ Methoxy-polyethylenglykolmonoacrylat-Copolymer;
Resorcin, Glyoxylsäure, AMPS/ Methoxy-polyethylenglykolmonoacrylat-Copolymer;
Phloroglucin, Glutardialdehyd, AMPP/Polyethylenglykolmonomethacrylat-Copolymer;
Phloroglucin, Succindialaldehyd, AMPP/Polyethylenglykolmonomethacrylat-Copolymer;
Phloroglucin, Glyoxal, AMPP/ Polyethylenglykolmonomethacrylat-Copolymer;
Phloroglucin, Glyoxylsäure, AMPS/Polyethylenglykolmonomethacrylat-Copolymer;
Phloroglucin, Glutardialdehyd, AMPP/Polyethylenglykolmonoacrylat-Copolymer;
Phloroglucin, Succindialaldehyd, AMPP/Polyethylenglykolmonoacrylat-Copolymer;
Phloroglucin, Glyoxal, AMPP/ Polyethylenglykolmonoacrylat-Copolymer;
Phloroglucin, Glyoxylsäure, AMPP/ Polyethylenglykolmonoacrylat-Copolymer;
Phloroglucin, Glutardialdehyd, AMPP/Polypropylenglykolmonomethacrylat-Copolymer;
Phloroglucin, Succindialaldehyd, AMPP/Polypropylenglykolmonomethacrylat-Copolymer;
Phloroglucin, Glyoxal, AMPP/ Polypropylenglykolmonomethacrylat-Copolymer;
Phloroglucin, Glyoxylsäure, AMPP/ Polypropylenglykolmonomethacrylat-Copolymer;
Phloroglucin, Glutardialdehyd, AMPP/Polypropylenglykolmonoacrylat-Copolymer;
Phloroglucin, Succindialaldehyd, AMPP/Polypropylenglykolmonoacrylat-Copolymer;
Phloroglucin, Glyoxal, AMPP/ Polypropylenglykolmonoacrylat-Copolymer;
Phloroglucin, Glyoxylsäure, AMPP/ Polypropylenglykolmonoacrylat-Copolymer;
Phloroglucin, Glutardialdehyd, AMPP/Methoxy-polyethylenglykolmonomethacrylat-Copolymer;
Phloroglucin, Succindialaldehyd, AMPP/Methoxy-polyethylenglykolmonomethacrylat-Copolymer;
Phloroglucin, Glyoxal, AMPP/ Methoxy-polyethylenglykolmonomethacrylat-Copolymer;
Phloroglucin, Glyoxylsäure, AMPP/ Methoxy-polyethylenglykolmonomethacrylat-Copolymer;
Phloroglucin, Glutardialdehyd, AMPP/Methoxy-polyethylenglykolmonoacrylat-Copolymer;
Phloroglucin, Succindialaldehyd, AMPP/Methoxy-polyethylenglykolmonoacrylat-Copolymer;
Phloroglucin, Glyoxal, AMPP/ Methoxy-polyethylenglykolmonoacrylat-Copolymer;
Phloroglucin, Glyoxylsäure, AMPP/ Methoxy-polyethylenglykolmonoacrylat-Copolymer.

Die erfindungsgemäß modifizierten Sprengmittel sind Verbindungen und Materialien, die bei einer Steigerung der Temperatur auf einen bestimmten Temperaturbereich expandieren oder Gase freisetzen, und so innerhalb der Kapsel den nötigen Druck aufbauen, die Schale der Mikrokapsel zu sprengen. Um einen hohen Druck aufzubauen ist es besonders vorteilhaft, Verbindungen zu verwenden, welche einen sehr niedrigen Siedepunkt besitzen oder die in einem definierten Temperaturbereich eine chemische Reaktion oder Zersetzung unter Freisetzung von Gasen eingehen. Dem Fachmann sind solche Verbindungen bekannt.

Es ist dabei vorteilhaft, das Sprengmittel so zu wählen, dass die Expansion, seine chemische Reaktion oder Zersetzung unter Freisetzen von Gasen in dem vorher definierten Temperaturbereich stattfindet.

Vorteilhafterweise öffnen mehr als 50%, vorzugsweise mehr als 70%, insbesondere bevorzugt mehr als 80% der Kapseln in einem Temperaturintervall von höchsten 20°C, vorteilhafterweise in einem Intervall von höchstens 10°C, insbesondere bevorzugt von höchsten 5°C. Damit wird der Wirkstoff aus den Mikrokapseln in einem gewünschten Temperaturbereich freigesetzt. Dieses ist beispielsweise bei dem Einsatz von Flammhemmern oder in chemischen Prozessen und Synthesen wünschenswert, wenn eine Freisetzung z.B. von Katalysatoren ab Erreichen einer bestimmten Temperatur notwendig oder wünschenswert ist. Ein weiterer vorteilhafter Effekt ist, dass ein Wirkstoff verkapselt an die Stelle seines Einsatzes verbracht werden kann und erst dort durch Erwärmen auf die Öffnungstemperatur freigesetzt werden kann. Ein weiterer Vorteil der erfindungsgemäßen Mikrokapsel besteht darin, dass stabile und dem Fachmann bekannte Polymere und Copolymere für die Schale der Mikrokapseln verwendet werden können Es wird somit ein System geschaffen, das sich bei gleichbleibendem Wandmaterial für Sprengmittel an die Anwendung anpasst; und nicht das Wandmaterial. Das bedeutet eine hohe Flexibilität bei niedrigen Kosten. Durch die Wahl geeigneter Sprengmittel, die in oberflächenmodifizierter Form nach der Erfindung, in die Mikrokapseln eingebracht sind, führen dazu, dass die Mikrokapseln in einer besonderen Ausführungsform der Erfindung bereits bei einer Temperatur von unter 150°C, vorzugsweise von unter 120°C, weiter bevorzugt von unter 100°C öffnen. Eine ganz besondere Ausführungsform der Erfindung ist eine Mikrokapsel, die in einem Temperaturbereich von 80-120°C oder 60-70°C öffnet. Die Mikrokapseln nach dem Stand der Technik, d.h. Mikrokapseln ohne Sprengmittel, öffnen dagegen häufig erst bei Temperaturen von etwa 200°C.

Sprengmittel im Sinne der vorliegenden Erfindung sind daher insbesondere auch Verbindungen, die bei Temperatureinwirkung Gase freisetzen sowie Partikel, die ein Treibmittel enthalten. Als Sprengmittel sind insbesondere Verbindungen geeignet, die Stickstoff ($N_2$) oder Kohlenstoffdioxid ($CO_2$) freisetzen. Auch andere Verbindungen, die bei Temperatureinwirkung in einem bestimmten Temperaturbereich nicht-toxische Gase wie Neon, Argon, dampfförmiges Wasser, niedermolekulare Kohlenwasserstoffe oder halogenierte Kohlenwasserstoffe freisetzen, eignen sich für die vorliegende Erfindung.

Insbesondere geeignet sind Natriumhydrogencarbonat, Natriumcarbonat, Kaliumcarbonat Kaliumhydrogencarbonat, Azodicarbonamide, Hydrazide wie beispielsweise p-Toluolsulfohydrazid, Carbazide wie beispielsweise 4,4-Oxy-bis(benzosulfohydrazid) und 2,2-Toluylensulfonylsemicarbazid,Tetrazole wie beispielsweise 5-Phenyltetrazol und/oder Zitronensäurederivate.

Daher ist in einer bevorzugten Ausführungsform der vorliegenden Erfindung das Sprengmittel der erfindungsgemäßen Mikrokapsel ausgewählt aus der Gruppe bestehend aus: Natriumhydrogencarbonat, Natriumcarbonat, Kaliumcarbonat Kaliumhydrogencarbonat, Azodicarbonamid, p-Toluolsulfohydrazid, 4,4-Oxy-bis(benzosulfohydrazid), 2,2-Toluylensulfonylsemicarbazid, 5-Phenyltetrazol und/oder Zitronensäurederivate.

In Tabelle 1 sind beispielhaft einige der besonders vorteilhaften Sprengmittel, ihre Zersetzungstemperaturen, ihre bei der Zersetzung gebildeten Gase sowie die Gasausbeute aufgeführt:

**Tabelle 1:** Verschiedene Sprengmittel.

| Bezeichnung | Zersetzungs-temp. (°C) | Gasausbeute (mL/g) | Gase |
|---|---|---|---|
| Azodicarbonamid | 200-300 | 280-320 | $N_2$, CO, ($NH_3$, $CO_2$) |
| p-Toluolsulfohydrazid | 110-140 | 120-140 | $N_2$, $H_2O$ |
| 4,4-Oxy-bis(benzosulfohydrazid) | 140-165 | 120-150 | $N_2$, $H_2O$ |
| 2,2-Toluylensulfonylsemicarbazid | 215-235 | 120-140 | $N_2$, $CO_2$ |
| 5-Phenyltetrazol | 240-250 | 190-210 | $N_2$ |
| Natriumhydrogencarbonat | 120-150 | 130-170 | $CO_2$, $H_2O$ |
| Zitronensäurederivate | 200-220 | 90-120 | $CO_2$, $H_2O$ |

Partikel, die sich als Sprengmittel im Sinne der vorliegenden Erfindung besonders eignen, enthalten ein Treibmittel, expandieren durch Temperaturerhöhung und bestehen vorzugsweise im wesentlichen aus einer Polymerhülle und einem gasförmigen oder flüssigen Kern, vorzugsweise bestehen sie aus einer Polymerhülle und einem Kern. Dabei kann das Polymer ein Polymer oder Copolymer sein. Beispiele für geeignete Polymere sind die dem Fachmann bekannten Polyethylen (PE), Polyurethan (PU), Polypropylen (PP), Polyvinylchlorid (PVC), Polymethylmethacrylat (PMMA), Polyvinylidendichlorid (PVDC), Polivinylidendichlorid-Acrylnitril-Copolymer und Poly(meth)acrylat. Geeignete gasförmige expandierbare Treibmittel sind Stickstoff, Kohlenstoffdioxid sowie niedermolekulare Kohlenwasserstoffe und niedermolekulare halogenierte Kohlenwasserstoffe. Beispiele für geeignete flüssige expandierbare Treibmittel sind niedermolekulare Kohlenwasserstoffe und niedermolekulare halogenierte Kohlenwasserstoffe mit einem niedrigen Siedepunkt im Bereich von 40 bis 150 °C, bevorzugt 50 bis 130 und noch mehr bevorzugt im Bereich von 60 bis 120 °C.

Die durchschnittliche Größe solcher expandierbarer Partikel liegt zwischen 5 und 50 $\mu$m, bevorzugt zwischen 10 und 40 $\mu$m und besonders bevorzugt zwischen 15 und 35 $\mu$m. Das Volumen der Partikel kann bevorzugt bei Expansion um das 50 fache bis 100 fache wachsen.

Eine bevorzugte Ausgestaltung der Sprengmittel gemäß der Erfindung sind expandierbare Partikel, umfassend eine Hülle aus Polymethylmethacrylat (PMMA) und/oder einem Alkylpolyacrylat, mit einer Wanddicke von 2 bis 15 Mikrometern, einem Kern aus einem flüssigen oder gasförmigen Kohlenwasserstoff mit einem Siedepunkt im Bereich von 40 bis 150 °C, bevorzugt 50 bis 130 und noch mehr bevorzugt im Bereich von 60 bis 120 °C und einem Durchmesser der Mikrokapsel im Bereich von zwischen 5 und 50 $\mu$m, bevorzugt zwischen 10 und 40 $\mu$m und besonders bevorzugt

zwischen 15 und 35 μm.

Geeignete Partikel dieser Art sind kommerziell erhältlich und können beispielsweise unter dem Handelsnamen Expancel von AkzoNobel sowie unter dem Handelsnamen Matsumoto Microspheres (F und FN series) von Matsumoto Yushi-Seiyaku Co., Ltd. (Japan) bezogen werden.

Erfindungsgemäß werden die Sprengmittel für den Verkapselungsprozess - also für die *in situ* Herstellung der Mikrokapseln - zuvor oberflächenmodifiziert. Damit ist es möglich, das Sprengmittel effizient zusammen mit dem Kernmaterial und dem Wirkstoff zu verkapseln.

In einer bevorzugten Ausführungsform der vorliegenden Erfindung ist das Sprengmittel so modifiziert, dass es ein zeta-Potential im Bereich von -0,9 bis 0,8 mV, bevorzugt im Bereich von -0,9 bis -0,01 mV oder im Bereich von 0,01 bis 0,8 mV oder im Bereich von -0,3 bis 0,4 mV aufweist. Das Zeta-Potential wird als elektrophoresische Moliblität gemessen (siehe unten).

Die Bestimmung der Oberflächenmodifizierung des zu verkapselnden Sprengmittels kann über das dem Fachmann bekannte zeta-Potential erfolgen. Das zeta-Potential (ζ-Potential) beschreibt das elektrische Potential (auch als Coulomb-Potential bezeichnet) an der Abscherschicht eines bewegten Partikels in einer Suspension. Das elektrische Potential beschreibt die Fähigkeit eines von der Ladung hervorgerufenen Feldes, Kraft auf andere Ladungen auszuüben. Das zeta-Potential gibt Aufschluss über den Grad der Abstoßung zwischen benachbarten Partikeln ähnlicher Ladung in einer Suspension.

Das zeta-Potential kann nicht direkt gemessen werden, sondern kann anhand theoretischer Modelle und der experimentell bestimmten elektrophoretischen Mobilität oder der dynamischen elektrophoretischen Mobilität (EM) berechnet werden. Ein Beispiel für die Bestimmung der elektrophoretischen Mobilität ist unten beschrieben.

Die Elektrophorese dient zur Abschätzung von zeta-Potentialen von Teilchen, wobei ein Strömungspotential/Strom für poröse Körper und flache Oberflächen verwendet wird. Die EM wird üblicherweise bestimmt, indem ein elektrisches Feld an eine Dispersion angelegt wird. Partikel innerhalb der Dispersion mit einem zeta-Potential bewegen sich dann zur entgegengesetzt geladenen Elektrode mit einer Geschwindigkeit, die proportional zur Größenordnung der EM ist und somit den Rückschluss auf das zeta-Potential erlaubt. Die Geschwindigkeit wird üblicherweise unter Verwendung eines Laser-Doppler-Anemometers gemessen. Der Frequenzverschiebung oder die Phasenverschiebung eines anregenden Laserstrahls durch die sich bewegenden Partikel wird gemessen als die Partikelmobilität, welche dann mit der Viskosität des Dispersionsmittels und der dielektrischen Permitiviät in das zeta-Potential umgerechnet wird.

Für den Einsatz in den üblichen Öl-in-Wasser-Verkapselungen ist es vorteilhaft, die Oberfläche der Sprengmittel zu hydrophobisieren, d.h. dergestalt auszurüsten, dass diese Oberfläche entsprechend dem hydrophoben Wirkstoff des Kernmaterials ebenfalls hydrophob ist, damit das Sprengmittel mit dem bzw. den hydrophoben Wirkstoff(en) zu den erfindungsgemäßen Mikrokapseln verkapselt werden kann. Die Oberflächenbehandlung führt somit zu einer Kompatibilität von Sprengmitteln und Kernmaterial. Diese Kompatibilität erlaubt eine effektive Verkapslung. In einer bevorzugten Ausführungsform der vorliegenden Erfindung ist daher die Oberfläche des Sprengmittels hydrophobisiert.

Eine erfindungsgemäße Oberflächenmodifizierung, d.h eine Hydrophobisierung der Oberfläche des Sprengmittels und damit die Einstellung eines bestimmten zeta-Potentials, wird vorzugsweise dadurch erreicht, dass die Oberfläche des Sprengmittels mit mindestens einer Verbindung aus der Gruppe bestehend aus Polyethylenimiden, quartären Ammoniumverbindungen, quartären Polyvinylpyrrolidonen und Ölsäure sowie Zubereitungen davon in organischen Lösungsmitteln wie z.B. n-Butanol, 1,4-Butandiol, Ethylenglycol oder Wasser behandelt wird.

In einer weiteren bevorzugten Ausführungsform der vorliegenden Erfindung ist die Oberfläche des erfindungsgemäß eingesetzten Sprengmittels mit einer Verbindung, ausgewählt aus der Gruppe bestehend aus Polyethylenimiden, quartären Ammoniumverbindungen, quartären Polyvinylpyrrolidonen und Ölsäure hydrophobisiert. Beispiele für quartäre Ammoniumverbindungen sind Betain, Cholinchlorid, Benzalkoniumchlorid sowie Didecyldimethylammoniumchlorid. Auch kommerziell erhältliche Ammoniumverbindungen sowie Zubereitungen von Ammoniumverbindungen können in der vorliegenden Erfindung vorteilhaft zur Modifkation der Sprengmitteloberflächen eingesetzt werden. Beispiele dafür sind Lanco Stat L 80 N, Lanco Stat LI 100, Lando Stat PUN und Lanco Stat FN der Lubrizol Deutschland GmbH.

Geeignete Polyethylenimid-Verbindungen sind multifunktionelle kationische Polymere auf Ethylenimid-Basis mit Molmassen im Bereich von 600 und 2500000 Da. Solche Polyethylenimide und Zubereitungen davon sind beispielsweise unter dem Handelsnamen Lupasol von der BASF SE (Deutschland) kommerziell erhältlich.

Quartäre Polyvinylpyrrolidone, die ebenfalls vorteilhaft in der vorliegenden Erfindung eingesetzt werden können, sind beispielsweise unter dem Handelsnamen Luviquat von der BASF SE (Deutschland) erhältlich.

In einer besonderen Ausführungsform der Erfindung beträgt der Anteil an den oberflächenbehandelten, hydrophobierten Sprengmitteln, zwischen 0,1 und 90 Gew.-% des Kernmaterial.

In einer bevorzugten Ausführungsform der vorliegenden Erfindung beträgt der Anteil der oberflächenbehandelten, hydrophobierten Sprengmitteln an dem zu verkapselnden Kernmaterial zwischen 0,1 und 90, bevorzugt zwischen 10 und 80 und besonders bevorzugt zwischen 20 und 70 Gew.-%, bezogen auf das Gesamtgewicht des Kernmaterials der erfindungsgemäßen Mikrokapseln.

Die Mikrokapseln gemäß der vorliegenden Erfindung lassen sich vorzugsweise durch ein mehrstufiges Verfahren herstellen. Dabei wird zunächst die Oberfläche des oder der Sprengmittel erfindungsgemäß modifiziert. Dazu wird vorteilhafterweise das mindestens eine Sprengmittel in Wasser zusammen mit einem oder mehreren der oben genannten Modifizierungsmittel suspendiert. Alternativ dazu kann das Modifizierungsmittel auch einer wässrigen Suspension des Sprengmittels zugegeben werden oder das Sprengmittel kann in einer Lösung oder Suspension der Modifizierungsmittel suspendiert werden. Bevorzugt wird das Sprengmittel anschließend getrocknet. Vorzugsweise kann das oberflächenmodifizierte Sprengmittel abfiltert, abgenutscht oder mittels Zentrifugation aus der Suspension abgetrennt und bei einer geeigneten Temperatur und/oder im Vakuum getrocknet werden.

Das auf diese Weise erhaltene oberflächenmodifizierte Sprengmittel wird dann in dem Wirkstoff oder zusammen mit dem Wirkstoff des Kernmaterials dispergiert und anschließend in einem dem Fachmann bekannten Verfahren mit dem Schalenmaterial zu den erfindungsgemäßen Mikrokapseln verkapselt.

Besonders bevorzugte Verfahren zur Herstellung von Mikrokapseln sowie bevorzugte Mikrokapseln sind in der WO 2011/110368 A2, sowie in der WO 2009/015872 A1 und der WO 2010/102830 A2 offenbart.

Verfahren zur Herstellung Melamin-Formaldehyd-Kapseln sind dem Fachmann unter anderem aus EP-A-0415273, EP-A-0218887, EP-A-0026914 und WO-A-01/51197 bekannt. Offenbart ist daher ein Verfahren, umfassend die Schritte (i) Modifizieren, insbesondere Hydrophobisieren der Oberfläche eines Sprengmittels und (ii) Umsetzen des oberflächenmodifizierten Sprengmittels mit dem Kernmaterial, umfassend mindestens einen Wirkstoff, und einem Schalenmaterial zu den Mikrokapseln gemäß der vorliegenden Erfindung.

Die erfindungsgemäße Mikrokapsel lässt sich also durch eine geeignete Wahl von Sprengmittel und Oberflächenmodifizierung in einem vorher definierten Temperaturbereich zur Öffnung und somit zur gezielten Freisetzung der Wirkstoffe des Kernmaterials bringen. Sie eignet sich daher hervorragend zur gezielten Anwendung in der Freisetzung von Duftstoffen in der Textilindustrie, zur Freisetzung von Duftstoffen und/oder Haar- und/oder Hautpflegeprodukten in der Kosmetik, die Freisetzung von Katalysatoren in der Polymersynthese oder der chemischen Synthese, sowie der Freisetzung von Schmiermitteln in der Automobil- und Maschinenbauindustrie.

Ein weiterer Gegenstand der vorliegenden Erfindung ist die Verwendung von erfindungsgemäßen Mikrokapseln oder Mikrokapseldispersionen für die kontrollierte Freisetzung von Kernmaterialen, die hydrophil (z.B. Aromastoffe) oder hydrophob sein können. Die Kernmaterialien sind beispielsweise Wirkstoffe, die vorzugsweise ausgewählt sind aus der Gruppe der Duft- und Aromastoffe, Pestizide, Herbizide, Schmiermittel, Gleitmittel (z.B. fluorierte Kohlenwasserstoffe), Insektizide, antimikrobiellen Wirkstoffe, pharmazeutischen Wirkstoffe, kosmetischen Wirkstoffe (z.B. für Shampoo), Latentwärmespeicher (z.B. Wachse), Katalysatoren (z. B. organische Carbonate, organometallische Verbindungen, Metallocene und dergleichen), Selbstheilungsagenzien (z.B. Norbornen, Dicyclopentadien), Beschichtungssysteme wie Lacke (z.B. Duftlacke), Farbstoffe (z.B. für kohlefreie Durchschreibesysteme), hydrophobe Wachse, hydrophobe En-Komponenten oder hydrophobe Lösungsmittel.

Die erfindungsgemäßen Kapseln lassen sich außerdem für die Zudosierung von Katalysatoren oder Initiatoren zu Beschichtungsmassen oder Tränkharzen einsetzen. Während der Imprägnierung oder Beschichtung von Substraten kommt es in Abhängigkeit von der Temperatur bei diesen zugesetzten reaktiven Bestandteilen zu Reaktionen, die z.B. die Viskosität der Tränkbäder stark verändern. Da oftmals nachfolgende thermische Trocknungsschritte oder thermische Vernetzungsreaktionen gewünscht sind, ist es besonders gewünscht, dass diese Katalysatoren in ausreichender Menge bei bestimmten Temperaturen zur Verfügung gestellt werden. Die gezielt thermisch öffnenden Kapseln nach der Erfindung führen daher zur besseren Steuerung und Dosierung, zur Herstellung neuartiger Polymerketten bei Initiator gestarteten Polymerisationen oder zur Nutzung farbgebender oder wirkstofffreisetzender Systeme unter gezielter Temperatureinwirkung. Diese Vorgehensweise ist umfassend einsetzbar, da Temperaturerhöhung und -einstellung den wichtigsten Verfahrensschritt in der Produktionstechnologie darstellt.

Es sind aber auch beliebige andere Stoffe möglich, die mit dem gewählten Mikrokapselsystem verkapselbar sind. Diese Stoffe werden im Kontext der vorliegenden Erfindung zusammenfassend als "Wirkstoffe" bezeichnet.

Somit kann der Wirkstoff mit dem Kernmaterial identisch sein.

Dieses ist in der chemischen Industrie von erheblicher Bedeutung. So werden beispielsweise bei der Herstellung von Polyurethan sowie bei der Herstellung von PU-Schäumen zurzeit Katalysatoren zugesetzt, die schon bei Raumtemperatur eine beträchtliche Aktivität entwickeln. Dieses beeinflusst die Herstellung entscheidend, da die Viskosität der Reaktionsmischungen in den verwendeten Reaktoren oder Tränkwannen stark ansteigt. Ein Katalysator, der erst in einem gewünschten Temperaturbereich aktiv wird, oder beispielsweise erst bei der Trocknung derart hergestellter Materialien freigesetzt wird, ist allerdings noch nicht bekannt und stark wünschenswert. Durch die Verwendung der erfindungsgemäßen oberflächenmodifizierten Sprengmitteln und durch die Verwendung der erfindungsgemäßen Mikrokapseln werden solche Katalysatoren unmittelbar bereitgestellt. Damit können bei der Herstellung von Polymersystemen unter Verwendung von in den erfindungsgemäßen Mikrokapseln verkapselten Initiatoren neue Prozessprofile und Eigenschaftsprofile der Produkte erhalten werden, die die Nutzung von Initiatoren, Katalysatoren, Mediatoren, Blockiermitteln, Kettenabbruchreagentien, Sensibilisatoren usw. ermöglichen. Die genannten Inhaltsstoffe werden erst bei einer vorher definierten Temperatur freigesetzt und beeinflussen die Reaktionen nicht vor der erfindungsgemäß thermisch ausgelösten Freisetzung.

Die Katalysatoren, die in den erfindungsgemäßen Mikrokapseln vorteilhafterweise verwendet bzw. verkapselt werden können, sind allgemein dem Fachmann bekannt. Vorteilhaft können insbesondere metallorganische Verbindungen verkapselt werden. Dieses sind metallorganische Verbindungen der Haupt- und Nebengruppenelemente mit einem oder mehreren Metallkernen wie zum Beispiel Triethylaluminium, Organostannane und dergleichen. Auch Ziegler-Natta-Katalysatoren oder Metallocene können vorteilhafterweise mit den erfindungsgemäßen Verfahren verkapselt werden. Weitere metallorganische Katalysatoren sind dem Fachmann bekannt und können in der vorliegenden Erfindung vorteilhaft verwendet werden. Offenbart ist daher eine Mikrokapsel, umfassend ein oberflächenmodifiziertes Sprengmittel wie oben definiert, einen metallorganischen Katalysator, bevorzugt Triethylaluminium, Organostannane, Ziegler-Natta-Katalysatoren oder Metallocene sowie ein Schalenmaterial wie oben definiert.

Außerdem offenbart sind Produkte, die erfindungsgemäße Mikrokapseln oder Mikrokapseldispersionen enthalten und deren Verwendung vorzugsweise auf einem Anwendungsfeld liegt, das ausgewählt ist aus den Gebieten der Beschichtungen, wie kohlefreie Durchschreibesysteme, Beschichtung und Imprägnierung von Papieren und Sicherheitsmerkmalbeschichtung, der katalysatorgefüllten Mikrokapseln, Lacktechnologie wie Lackherstellung, Bauchemie, Dentaltechnik vorzugsweise als Bestandteil für schnell härtende Zahnfüllmassen, Selbstheilungssysteme, Kosmetik vorzugsweise für Duft- und Aromaöle, Pharmazie vorzugsweise als Wirkstoffträger, Medizintechnik, z.B. zur Verkapselung von Emittenten von Neurotransmittern wie NO, z.B. von Nitroglycerin, Waschen, Reinigen, Desinfizieren, Kleben, Flammschutz, der Behandlung von Pflanzen, vorzugsweise als Fungizid, Pestizid, Insektizid, Herbizid oder Korrosionsschutz.

Die erfindungsgemäßen Mikrokapseln lassen sich z.B. für die Herstellung von Lacken z.B. für Duftlacke einsetzen und sind in ihrem Vernetzungsgrad, ihrer Größe ihrer Wandstärke und Oberflächenausrüstung sowie im Kernmaterial variabel einsetzbar.

Aufgrund der hohen chemischen und physikalischen Resistenz eignen sie sich als stabile Kern/Schale Kapselsysteme, auch für den Einsatz in aggressiven Medien. So ist es möglich Duftlacke herzustellen, die über herkömmliche Rakelsysteme mit den in der Druckindustrie bekannten Schichtstärken zu beschichten, ohne dass ein nennenswerter Anteil der Kapseln zerstört wird.

Die Mikrokapseln weisen im Allgemeinen einen mittleren Durchmesser von 1-1000 $\mu$m auf. Im Rahmen der vorliegenden Erfindung sind jedoch vom Begriff Mikrokapsel auch Nanokapseln umfasst, d.h. Kapseln mit einem mittleren Durchmesser < 1 $\mu$m. Die Kapseln weisen vorzugsweise einen mittleren Durchmesser von 0,1 bis 100 $\mu$m auf. Die Wandstärke ist einstellbar und kann 0,01-100 $\mu$m, insbesondere zum Beispiel 0,1 bis 10 $\mu$m betragen.

Die erfindungsgemäßen Mikrokapseln können in Form wässriger Dispersionen als Tränkharze im Holz/Werkstoff-Bereich eingesetzt werden und eignen sich insbesondere als Imprägnierharze mit zusätzlichen Funktionen wie katalytische Effekte, Farbeffekte, thermochrome Effekte oder Sicherheitseffekte für dekorative Beschichtungssysteme.

Ein weiterer Gegenstand der vorliegenden Erfindung ist die Verwendung der erfindungsgemäßen Mikrokapseln zur Freisetzung von Duftstoffen in der Textilindustrie, zur Freisetzung von Duftstoffen und/oder Haar- und/oder Hautpflegeprodukten in der Kosmetik, die Freisetzung von Katalysatoren in der Polymersynthese oder der chemischen Synthese, und der Freisetzung von Schmiermitteln in der Automobil- und Maschinenbauindustrie.

**Beschreibung der Figuren:**

Die Figuren zeigen beispielhaft die thermische Öffnung von erfindungsgemäßen Mikrokapseln mit einer Melamin-Formaldehyd-Schale. Es werden Mikroskop-Aufnahmen einer Dispersion der Mikrokapseln in einem durchsichtigen, farblosen Öl bei verschiedenen Temperaturen gezeigt, um die Öffnung der Mikrokapseln in einem bestimmten Temperaturbereich zu verdeutlichen.

**Figur 1**: Aufnahme bei 25 °C. Die kugelförmigen dunklen Mikrokapseln sind noch unbeschädigt vor dem hellen Hintergrund deutlich zu erkennen.

**Figur 2**: Aufnahme bei 75 °C: Die Mehrzahl der Mikrokapseln ist noch unbeschädigt. Die beginnende Öffnung einiger Mikrokapseln ist anhand der hellen, kreisförmigen Bereiche in der Dispersion gut zu erkennen.

**Figur 3**: Aufnahme bei 85 °C: Die Öffnung einer Vielzahl der Mikrokapseln ist nun deutlicher zu erkennen als noch bei der Aufnahme bei 75 °C.

**Figur 4**: Apparatur zum Überprüfen des Öffnungstemperaturbereichs von Mikrokapseln mit Sprengmitteln wie in Beispiel 4 beschrieben. In der Figur 4 bedeuten

Bezugsziffer 1: Thermofühler
Bezugsziffer 2: Reagenzglas mit Füllgut (Prüfmaterial)

Bezugsziffer 3: Becherglas mit Themoöl
Bezugsziffer 4: Magnetfisch
Bezugsziffer 5: Heizplatte mit Magnetrührer

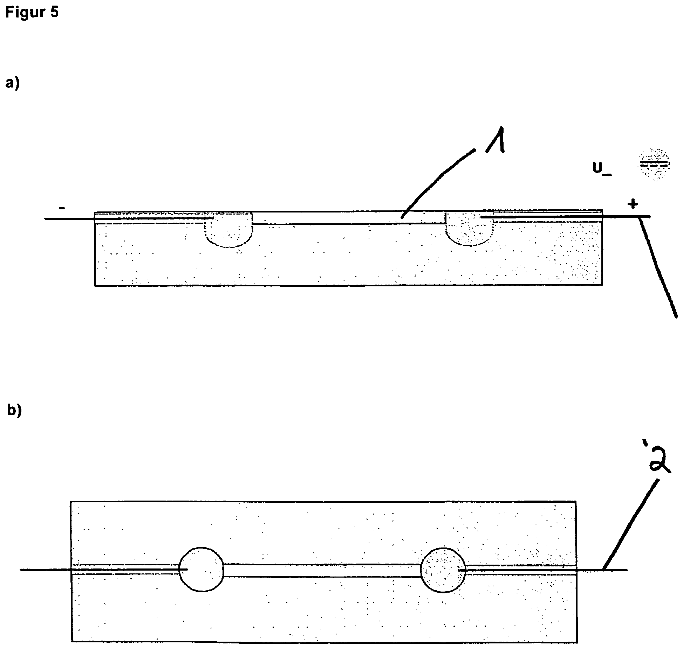

**Figur 5**: Darstellung einer Vorrichtung zur Messung der elektrophoretischen Mobilität (EM) als Maß für das zeta-Potential. In der Figur 5a (Seitenansicht) bedeutet die Bezugsziffer 1: Glaskapillar und in der Figur 5b (Aufsicht) bedeutet die Bezugsziffer 2: Platinelektroden.

Im Folgenden soll die vorliegende Erfindung durch Beispiele verdeutlicht werden. Dabei sind die Beispiele keineswegs limitierend für den beanspruchten Gegenstand der vorliegenden Erfindung, sondern dienen lediglich der Veranschaulichung der Erfindung für den Fachmann.

**Beispiel 1: Herstellung eines oberflächenmodifizierten Sprengmittels**

Es wurde aus Benzalkoniumchlorid und demineralisiertem Wasser eine 10%ige wässrige Lösung von Benzalkoniumchlorid hergestellt. Als Sprengmittel wurden 600 g Expancel 461DU (ex AkzoNobel) in 400 g demineralisertem Wasser (Verhältnis Expancel:Wasser 1:1) bei einer Rührgeschwindigkeit von 2500 RPM über 5 min suspendiert und anschließend derart mit der wässrigen Benzalkoniumchlorid-Lösung unter Rühren versetzt, dass der Gewichtsanteil des Benzalkoniumchlorides 1,5 % vom Gewicht des Expancels betrug. Die auf diese Weise erhaltene wässrige Dispersion wurde abgenutscht, zweimal mit 300 g demineralisiertem Wasser gespült und der so erhaltene Filterkuchen auf Filterpapier 24 Stunden bei 45 °C getrocknet. Anschließend konnte der Filterkuchen in die oberflächenmodifizierten Expancel-Mikrospheres zerkleinert werden. Die so erhaltenen oberflächenmodifizierten Sprengmittel konnten direkt in Öl (ND 100 Gleitmittel homogen verteilt werden. Zur Öl/Sprengmittel-Dispersion wurde demineralisiertes Wasser gegeben und nach mehrfachen Schütteln überprüft, in welcher Phase sich die oberflächenmodifizierten Sprengmittel konzentrierten. Es zeigte sich, dass die oberflächenmodifizierten z.B. Expancel-Microspheres in der Ölphase, aber nicht in der wässrigen Phase zu finden waren. Damit wurden Sprengmittel erhalten, deren Oberfläche hydrophobisiert worden ist.

**Vergleichsbeispiel 1: Sprengmittel ohne Oberflächenmodifizierung**

Expancel 461 DU (ex AkzoNobel) wurde in Öl (ND 100) gegeben und anschließend demineralisiertes Wasser hinzugefügt. Nach mehrfachen Schütteln wurde überprüft, in welcher Phase sich die Expancel-Microspheres konzentrierten. Es zeigte sich, dass die Microspheres sich durchgehend in beiden Phasen befanden, was für eine Verkapselung zusammen mit einem hydrophoben Wirkstoff (etwa einem Öl) nachteilig ist.

**Beispiel 2: Sprengmittel mit Oberflächenmodifizierung**

Die oberflächenmodifizierten Expancel 461 DU-Sprengmittel wurden zu je 10 % in C14-C17 Paraffin (Linpar C14-17 ex Sasol) eingearbeitet und ca. 5 mL dieser Mischung in ein dünnwandiges Reagenzglas gefüllt. Dieses Reagenzglas wurde in ein mit PEG 400 gefülltes Becherglas als Ölbad auf einer mit einem Thermofühler gesteuerten Magnetheizplatte zentriert positioniert. Der Meniskus der Sprengmittel-Paraffin-Dispersion wurde auf die Höhe des Meniskus des Ölbades eingestellt und dieses bei einer Heizrate von ca. 2°C/min mit einem Magnetrührer umgewälzt, um eine gleichmäßige Temperaturverteilung und eine gleichmäßige Heizrate zu gewährleisten. Die Heizrate betrug ca. 2 °C/min im erwarteten Expansionsbereich der oberflächenmodifizierten Expance-Partikel.

Die Expansion begann bei 94 °C und erreichte bei 96 °C ihr Maximum. Diese Werte entsprechen unbehandelten Expancel 461 DU-Partikel. Somit hat die Oberflächenbehandlung gemäß der vorliegenden Erfindung keinen Einfluss auf die Expansionstemperatur.

Herstellung stabiler Kern/Schale-Mikrokapseln mit Wirkstoff und oberflächenmodifizierten Sprengmitteln:

**Beispiel 3: Phloroglucin-Melamin Mikrokapsel**

a) Herstellung des Vorkondensats

5,4 g Phloroglucin und 0,6 g Melamin werden in 78,6 g destilliertem Wasser dispergiert. Der pH-Wert wird mit 1,2 g 85%iger Ameisensäure auf 3 eingestellt. Es wird auf 35 °C erwärmt und 14,2 g 50%ige Glutaraldehydlösung zugegeben. Nach 5 min beginnt sich das lösliche Vorkondensat zu bilden, erkennbar daran, dass das in Wasser kaum lösliche Phloroglucin und Melamin sich auflöst. Der Gesamtfestkörper des Vorkondensats beträgt 14,0 Gew.-%

b) Herstellung der Mikrokapsel

41,5 g des in Stufe a) erhaltenen löslichen Vorkondensats werden nach 5 min mit 3,0 g des Schutzkolloids, einem Copolymer aus AMPS (2-Acrylamido-2-methyl-1-propylsulfonsäure und PEM 6 (Polyethylenglykolmonomethacrylat) und 23,7 g eines zu verkapselnden Duftöls (Radiance) und des nach Beispiel 1 erhaltenen Sprengmittel (Verhältnis Duftöl:Sprengmittel = 70:30) versetzt. Zur Teilchenbildung wird gleichzeitig die Drehzahl von 500 U/min auf 2500 U/min erhöht. Nach 20 min beginnt das Harz zu strukturierten Kapselwänden auszuhärten. In der folgenden Stunde wird bei einer Umdrehungszahl von 600 U/min gerührt. Innerhalb dieser Stunde wird nach 15 min 7,5 g einer auf pH 3 mit Ameisensäure (85 %) angesäuerten 14 gew.-%igen Phloroglucin-Schlämme 45 min lang hinzudosiert, sowie nach 20 min 16 g Wasser hinzugegeben, um ein Dickwerden der Slurry zu vermeiden. Darauf folgt eine 2-stündige Aushärtungsphase bei 80 °C. Anschließend wird 4,2 g einer mit Ameisensäure 85 % angesäuerten, 33 gew.%igen Melamin-Schlämme (Folco-Schlämme) eine ½ h zudosiert. Es wird schließlich ½ h bei pH- 3 nachgehärtet. Die Kapselslurry wird auf Raumtemperatur abgekühlt und mit Natronlauge auf pH 7 eingestellt.

Technische Daten der erhaltenen Mikrokapsel:

| | |
|---|---|
| Durchmesser D(90): | 10μm |
| Festkörper: | 33 % |
| Kernanteil: | 70 % |
| Effizienz: | 90 % |
| Pulverausbeute: | 90% |
| Restaldehydgehalt: | < 500 ppm, ermittelt mit GC (FT-IR) |

Eine Probe der so erhaltenen Mikrokapseln wurde in farbloses Öl dispergiert und mittels eines Mikroskopes überprüft, ob noch Expancel-Partikel außerhalb der erfindungsgemäßen Mikrokapseln zu finden waren. Es zeigte sich, dass außerhalb der erfindungsgemäßen Mikrokapseln keine Expancel-Partikel sichtbar waren. Damit konnte gezeigt werden, dass die Herstellung von Mikrokapseln aus einem hydrophoben Öl, den erfindungsgemäßen oberflächenmodifizierten Sprengmitteln und den Grundstoffen für die Kapselhülle gemäß der vorliegenden Erfindung erfolgreich war.

**Beispiel 4: Überprüfung der thermischen Öffnung der Mikrokapseln**

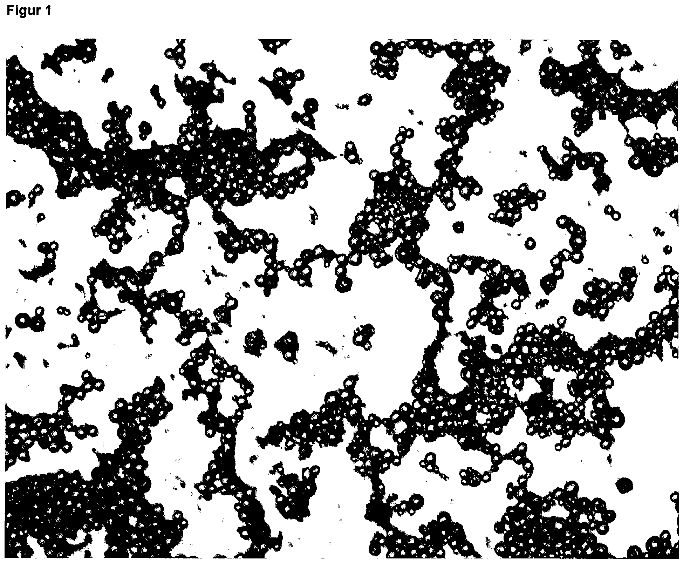

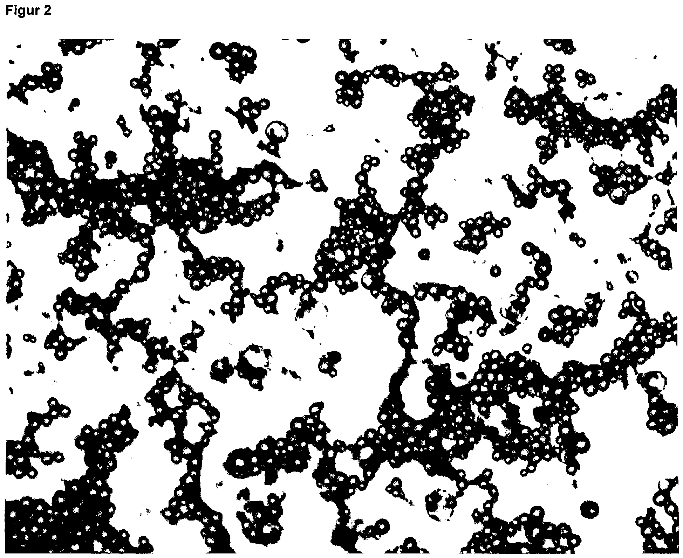

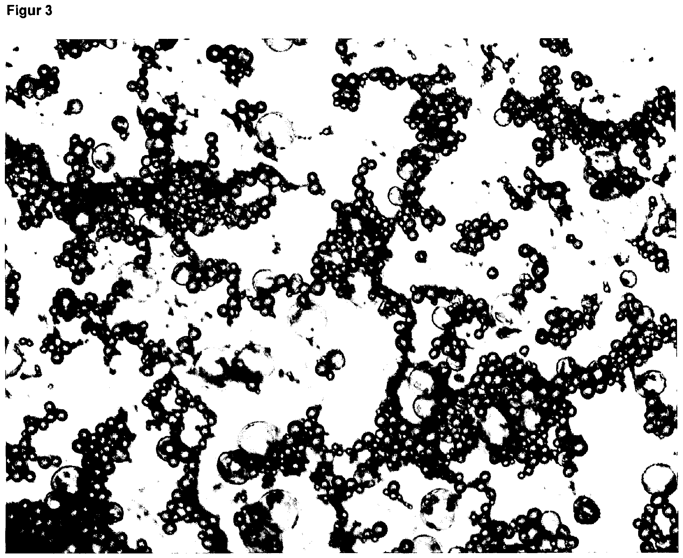

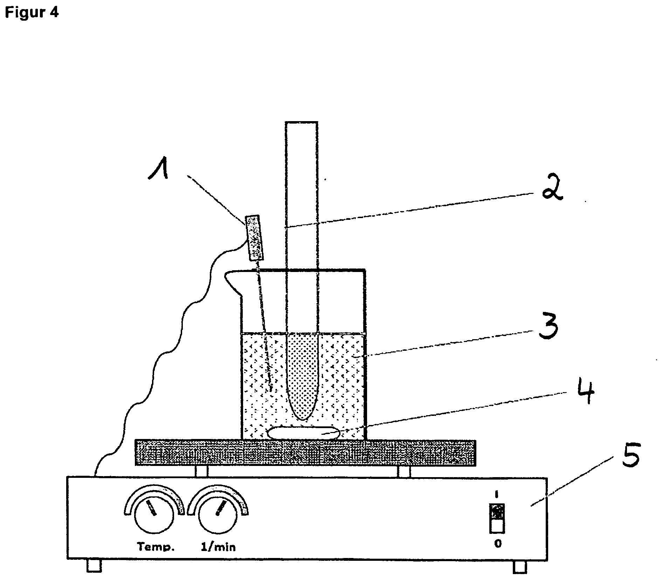

Die in Beispiel 3 erhaltene Öldispersion der erfindungsgemäßen Mikrokapseln wurden in einem Reagenzglas in eine Apparatur wie in Figur 4 gezeigt gegeben und mittels der Heizplatte eine Heizrate von ca. 2 °C/min erzeugt. Über ein Mikroskop wurde dann die Öffnung der Mikrokapseln bei verschiedenen Temperaturen überprüft. Erwartungsgemäß zeigte sich bei Raumtemperatur noch keine Öffnung, erst bei steigenden Temperaturen im durch die Expansionstemperatur vorgegebenen Öffnungsbereich konnte dann wie in den Figuren 1 bis 3 gezeigt die Öffnung der erfindungsgemäßen Mikrokapseln beobachtet werden.

**Beispiel 5 : Anwendungsbeispiel A**

Die in Beispiel 3 erhaltenen Mikrokapseln wurden in einen Lack überführt und auf einer Papieroberfläche aufgebracht. Auf diese Weise wurde eine Oberfläche erzeugt, die beim Einwirken einer bestimmten Temperatur einen Duft freisetzt. Damit sind die erfindungsgemäßen Mikrokapseln beispielsweise hervorragend für Anwendungen in der Werbebranche, der Kosmetik-, Körperpflege und Parfümindustrie geeignet.

**Beispiel 6: Anwendungsbeispiel B**

Analog der Verfahrensweise des Beispiels 3 wurde Dimethylzinn-neodecanoat (Fomrez tin catalyst UL-28, bezogen von Momentive performance materials) mit 30 % Expancel DU 40 als Sprengmittel, das mit 0,5 % Lupasol PS oberflächenmodifiziert worden war, zum einen in Melamin-Formaldehyd-Mikrokapseln und zum anderen in Phloroglucin-Glutaraldehyd-Kapseln verkapselt.

Die Kapseln wurden wie in Beispiel 3 auf ihre Verkapselung und wie in Beispiel 4 auf ihre thermische Öffnung überprüft. Sowohl bei den Formaldehyd-Melamin-Mikrokapseln als auch bei den Phloroglucin-Glutaraldehyd-Mikrokapseln wurde eine Verkapselung des Katalysators und des Sprengmittels beobachtet. Beide Mikrokapsel-Systeme öffneten bei der erwarteten Temperatur und setzten den Katalysator frei.

Damit wurde ein verkapselter Katalysator für die Polyurethan-Synthese (PU-Synthese) bereit gestellt, der erst in einem engen und genau definierbaren Temperaturbereich freigesetzt wird. Dieses ist äußerst vorteilhaft bei der Planung der Herstellung von PU bzw. PU-Produkten wie Beschichtungen, Schäume und dergleichen. Dadurch wird ebenfalls die ausgezeichnete Eignung der Mikrokapseln der vorliegenden Erfindung für die gezielte Freisetzung von Katalysatoren und anderen Stoffen in der chemischen Synthese und in chemischen Industrieverfahren deutlich.

**Beispiel 7: Bestimmung der elektrophoretischen Mobilität**

Zur Bestimmung der elektrophoretischen Mobilität und dem daraus errechenbarem Zeta-Potential wurden verschieden Meßzellen aufgebaut um zu bestimmen unter welchen Bedingungen sich reproduzierbare Ergebnisse ermitteln lassen. Grundlage zum beschriebenen Verfahren ist, dass sich Partikel in einem elektrischen Feld, gemäß ihrer Oberflächenladung mit einer bestimmten Geschwindigkeit zur entsprechenden Elektrode bewegen (positiv geladene Teilchen zur negativ geladenen Elektrode). Dieses Grundprinzip wird z.B. bei der Elektrophorese und seit einiger Zeit auch für die Zeta-Potentialmessung verwendet. Ziel der Versuche sollte es sein ein für unseren Anwendungsbereich (Mikrokapseln/Microspheres im Teilchengrößenbereich von $> 5 \,\mu m$ bis $< 40 \,\mu m$) geeignetes Meßverfahren zu entwickeln, mit dem vergleichende Messungen möglich werden (z.B. Erkennung von Trends bzgl. der Kationisierung).

Zur Messung wird das Prüfmaterial mit einer geeigneten Flüssigkeit gemischt und in eine der beiden Vertiefungen in der Meßzelle gefüllt. Aufgrund der Kapillarwirkung wird das Röhrchen vollständig geflutet. Ist dies erfolgt, wird die zweite Kammer befüllt. Durch das hydrostatische Paradoxon (kommunizierende Röhren) stellt sich zwischen den Kammern ein gleichmäßiges Flüssigkeitsniveau ein. Dieser Vorgang dauert in der beschriebenen Konfiguration, je nach Viskosität des verwendeten Verdünnungsmittels, ca. 1 bis 10 Minuten. Erkennbar ist der vollständige Niveauausgleich durch Beobachtung der Kapillare mit dem Mikroskop. Wenn die Partikel in keine Richtung mehr fließen, kann mit der eigentlichen Messung begonnen werden.

Hierzu werden die Elektroden an der Spannungsquelle (Gleichspannungsschaltnetzteil 5 - 24 V) angeschlossen. Am Mikroskop wird eine geeignete Schärfeebene über der Kapillare eingestellt (100-fache Vergrößerung), so dass die Kapseln mit vertretbarer Schärfe abgebildet werden. In der Mikroskopiersoftware wird eine geeignete Zeitsequenz (Serienaufnahme) eingestellt. Danach wird die Spannungsquelle eingeschaltet und nach ca. 1 Minute die Sequenzaufnahme gestartet.

Nach der Aufnahme kann die Zeitsequenz abgespielt, durch Einzelbildbetrachtung ausgewählte Kapseln in ihrer Bewegung beobachtet und die zurückgelegte Strecke ausgemessen werden. Aus der zurückgelegten Strecke wird die Geschwindigkeit errechnet (cm/s). Außerdem wird die Bewegungsrichtung (Anode oder Kathode) erfasst.

Das zeta-Potential lässt sich, je nach Anwendungsfall u.a., nach folgender Formel berechnen:

$$\zeta = \frac{4\pi\eta}{\varepsilon} \times U \times 300 \times 300 \times 1000$$

$\zeta$ = Zeta Potential ($mV$)

$\eta$ = Viscosity of Solution

$\varepsilon$ = Dielectric Constant

$U = \dfrac{v}{V/L}$ : Electrophoretic Mobility

$v$ = Speed of Particle ($cm$ / sec)

$V$ = Voltage ($V$)

$L$ = The distance of Electorode

Weitere Formeln:

$$v = \mu e * E$$

(v = Wanderungsgeschwindigkeit [cm/s] ; $\mu e$ = elektrophoretische Mobilität; E = elektrische Feldstärke [V/m])

$$E = U / d$$

(elektrische Feldstärke [V/m] = Spannung[V] / Elektro

**Patentansprüche**

1.  Mikrokapsel, umfassend

    a) eine Schale
    b) einen Kern, umfassend mindestens einen Wirkstoff und mindestens ein oberflächenmodifiziertes Sprengmittel,

    wobei das Sprengmittel ein expandierbarer Partikel ist mit einer Partikelhülle und einem Partikelkern, **dadurch gekennzeichnet, dass** die Oberfläche der Partikelhülle hydrophobisiert ist.

2.  Mikrokapsel gemäß Anspruch 1, **dadurch gekennzeichnet, dass** die Oberfläche der Partikelhülle mit einer Verbindung ausgewählt aus der Gruppe der folgenden Verbindungen hydrophobisiert ist: Polyethylenimide, quartäre Ammoniumverbindungen, quartäre Polyvinylpyrrolidone und Ölsäure, bevorzugt Benzalkoniumchlorid und Didecyldimethlyammoniumchlorid, besonders bevorzugt Benzalkoniumchlorid.

3.  Mikrokapsel gemäß einem der Ansprüche 1 oder Anspruch 2, **dadurch gekennzeichnet, dass** sich die Mikrokapsel bei einer Temperatur von unter 150°C öffnet.

4.  Mikrokapsel gemäß mindestens einem der vorhergehenden Ansprüche, **dadurch gekennzeichnet, dass** die Schale der Mikrokapsel ein Material ausgewählt aus der Gruppe bestehend aus Melamin-Formaldehyd, Phloroglucin-Melamin und Phloroglucin-Glutaraldehyd aufweist.

5.  Mikrokapsel nach einem der vorherigen Ansprüche, **dadurch gekennzeichnet, dass** das Sprengmittel Stickstoff oder Kohlendioxid freisetzt.

6.  Mikrokapsel nach einem der vorherigen Ansprüche, **dadurch gekennzeichnet, dass** das Sprengmittel Natriumhydrogencarbonat, Natriumcarbonat, Kaliumcarbonat Kaliumhydrogencarbonat, Azodicarbonamid, p-Toluolsulfohydrazid, 4,4-Oxy-bis(benzosulfohydrazid), 2,2-Toluylensulfonylsemicarbazid, 5-Phenyltetrazol und/oder Zitronensäurederivate umfasst.

7.  Mikrokapsel nach einem der vorherigen Ansprüche **dadurch gekennzeichnet, dass** die Partikelhülle ein Polymer oder ein Co-Polymer ausgewählt aus der Gruppe folgender Verbindungen umfaßt: Polyethylen (PE), Polyurethan (PU), Polypropylen (PP), Polyvinylchlorid (PVC), Polymethylmethacrylat (PMMA), Polyvinylidendichlorid (PVDC), Polivinylidendichlorid-Acrylnitril-Copolymer und Poly(meth)acrylat.

8.  Mikrokapsel nach Anspruch 1 **dadurch gekennzeichnet, dass** die Partikelhülle aus Polymethylmethacrylat und der Partikelkern aus Kohlenwasserstoff besteht.

9.  Mikrokapsel nach einem der vorherigen Ansprüche, **dadurch gekennzeichnet, dass** das Sprengmittel ein zeta-Potential im Bereich von -0,9 bis 0,8 mV, bevorzugt im Bereich von -0,9 bis -0,01 mV oder im Bereich von 0,01 bis 0,8 mV oder im Bereich von -0,3 bis 0,4 mV aufweist.

10. Verwendung einer Mikrokapsel nach einem der Ansprüche 1 bis 9 zur Freisetzung von Duftstoffen, Aromastoffen in der Textilindustrie, zur Freisetzung von Duftstoffen/Aromastoffen und/oder Haar- und/oder Hautpflegeprodukten in der Kosmetik, zur Freisetzung von Katalysatoren in der Polymersynthese, zur Freisetzung zum Aufbau von PU-Klebstoffen, - Schaumstoffen, - Bindemitteln oder Beschichtungsmassen, zur Freisetzung von Katalysatoren in der chemischen Synthese, zur Freisetzung von Schmiermitteln in der Automobil- und Maschinenbauindustrie, zur Freisetzung von Farbstoffen, Pigmenten und Indikatoren für sicherheitsrelevante Anwendungen.

**Claims**

1.  Microcapsule, comprising

    a) a shell
    b) a core, comprising at least one active ingredient and at least one surface-modified disintegrant,

wherein the disintegrant is an expandable particle with a particle sheath and a particle core, **characterised in that** the surface of the particle sheath is hydrophobized.

2. Microcapsule according to claim 1, **characterised in that** the surface of the particle sheath is hydrophobized with a compound selected from the group of the following compounds: polyethylene imides, quaternary ammonium compounds, quaternary polyvinyl pyrrolidones and oleic acid, preferably benzalkonium chloride and didecyldimethlyammonium chloride, particularly preferably benzalkonium chloride.

3. Microcapsule according to one of the claims 1 or 2, **characterised in that** the microcapsule opens at a temperature of below 150°C.

4. Microcapsule according to at least one of the preceding claims, **characterised in that** the shell of the microcapsule contains a material selected from the group consisting of melamine formaldehyde, phloroglucine melamine and phloroglucine glutaraldehyde.

5. Microcapsule according to one of the preceding claims, **characterised in that** the disintegrant releases nitrogen or carbon dioxide.

6. Microcapsule according to one of the preceding claims, **characterised in that** the disintegrant comprises sodium hydrogen carbonate, sodium carbonate, potassium carbonate, potassium hydrogen carbonate, azodicarbonamide, p-toluenesulphonyl hydrazide, 4,4-oxy-bis(benzosulphohydrazide), 2,2-toluylenesulphonyl semicarbazide, 5-phenyltetrazole and/or citric acid derivatives.

7. Microcapsule according to one of the preceding claims, **characterised in that** the particle sheath comprises a polymer or a copolymer selected from the group of the following compounds: polyethylene (PE), polyurethane (PU), polypropylene (PP), polyvinylchloride (PVC), polymethylmethacrylate (PMMA), polyvinylidene dichloride (PVDC), polivinylidene dichloride-acrylnitrile copolymer and poly(meth)acrylate.

8. Microcapsule according to claim 1, **characterised in that** the particle sheath consists of polymethylmethacrylate and the particle core of hydrocarbon.

9. Microcapsule according to one of the preceding claims, **characterised in that** the disintegrant has a zeta potential in a range from -0.9 to 0.8 mV, preferably in a range from -0.9 to -0.01 mV or in a range from 0.01 to 0.8 mV or in a range from -0.3 to 0.4 mV.

10. Use of a microcapsule according to one of the claims 1 to 9 for releasing fragrances, aroma substances in the textile industry, for releasing fragrances/aroma substances and/or hair and/or skin care products in cosmetics, for releasing catalysts in a polymer synthesis, for releasing for constructing PU adhesives, PU foaming agents, PU binding agents or coating compounds, for releasing catalysts in a chemical synthesis, for releasing lubricants in the automotive and mechanical engineering industry, for releasing dyes, pigments and indicators for safety-related applications.

**Revendications**

1. Microcapsule comprenant

   a) une membrane
   b) un noyau, comprenant au moins un principe actif et au moins un moyen d'éclatement modifié en surface,

   où le moyen d'éclatement est une particule pouvant se déployer présentant une enveloppe de particule et un noyau de particule, **caractérisée en ce que** la surface de l'enveloppe de particule est rendue hydrophobe.

2. Microcapsule selon la revendication 1, **caractérisée en ce que** la surface de l'enveloppe de particule est rendue hydrophobe avec un composé sélectionné parmi le groupe constitué des composés suivants : des polyéthylèneimides, des composés d'ammonium quaternaire, des polyvinylpyrrolidones quaternaires et de l'acide oléique, de préférence du chlorure de benzalkonium et du chlorure de didécyldiméthylammonium, de façon davantage préférée, du chlorure de benzalkonium.

**3.** Microcapsule selon la revendication 1 ou la revendication 2, **caractérisée en ce que** la microcapsule s'ouvre à une température inférieure à 150 °C.

**4.** Microcapsule selon au moins une des revendications précédentes, **caractérisée en ce que** la membrane de la microcapsule est constituée d'un matériau sélectionné parmi le groupe constitué de mélamine-formaldéhyde, de phloroglucinol-mélamine et de phloroglucinol-glutaraldéhyde.

**5.** Microcapsule selon l'une des revendications précédentes, **caractérisée en ce que** le moyen d'éclatement libère de l'azote ou du dioxyde de carbone.

**6.** Microcapsule selon l'une des revendications précédentes, **caractérisée en ce que** le moyen d'éclatement contient du bicarbonate de sodium, du carbonate de sodium, du carbonate de potassium, du bicarbonate de potassium, de l'azodicarbonamide, du p-toluolsulfohydrazide, du 4,4-oxy-bis(benzosulfohydrazide), du 2,2-toluylènesulfonylsemicarbazide, du 5-phényltétrazol et/ou des dérivés de l'acide citrique.

**7.** Microcapsule selon l'une des revendications précédentes, **caractérisée en ce que** l'enveloppe de particule contient un polymère ou un copolymère sélectionné parmi le groupe constitué des composés suivants : polyéthylène (PE), polyuréthane (PU), polypropylène (PP), poly(chlorure de vinyle) (PVC), poly(méthacrylate de méthyle) (PMMA), poly(dichlorure de vinylidène) (PVDC), copolymère de poly(dichlorure de vinylidène)-acrylonitrile et poly(méth)acrylate.

**8.** Microcapsule selon la revendication 1, **caractérisée en ce que** l'enveloppe de particule est constituée de poly(méthacrylate de méthyle) et le noyau de particule est constitué un hydrocarbure.

**9.** Microcapsule selon l'une des revendications précédentes, **caractérisée en ce que** le moyen d'éclatement présente un potentiel zêta dans la plage de -0,9 à 0,8 mV, de préférence dans la plage de -0,9 à -0,01 mV ou dans la plage de 0,01 à 0,8 mV ou dans la plage de -0,3 à 0,4 mV.

**10.** Utilisation d'une microcapsule selon l'une des revendications 1 à 9 à des fins de libération de parfums, d'arômes dans l'industrie du textile, de libération de parfums/d'arômes et/ou de produits de soins capillaires et/ou cutanés dans l'industrie des cosmétiques, de libération de catalyseurs dans la synthèse de polymères, de libération en vue d'une synthèse de colles, de mousses ou de liants respectivement, à base de PU ou de produits de revêtement, de libération de catalyseurs dans la synthèse chimique, de libération de lubrifiants dans l'industrie de l'automobile et de la mécanique, de libération de colorants, de pigments et d'indicateurs pour des applications relatives à la sécurité.

Figur 1

**Figur 2**

**Figur 3**

**Figur 4**

**Figur 5**

a)

b)

**IN DER BESCHREIBUNG AUFGEFÜHRTE DOKUMENTE**

**In der Beschreibung aufgeführte Patentdokumente**

- KR 20050084965 A
- WO 2010014011 A
- JP 2005115194 A
- WO 2011110368 A
- WO 2011110368 A2
- WO 2009015872 A1
- WO 2010102830 A2
- EP 0415273 A
- EP 0218887 A
- EP 0026914 A
- WO 0151197 A

**In der Beschreibung aufgeführte Nicht-Patentliteratur**

- **W. WANG et al.** Microfluidic Preparation of Multicompartment Microcapsules for Co-Encapsulation and Controlled Release of Multiple Components, Poster-abstract. Minneapolis Convention Center, 19. Oktober 2011